# EUROPÄISCHE PATENTANMELDUNG

(11) **EP 1 231 203 A2**
(43) Veröffentlichungstag der Anmeldung: **14.08.2002**
(21) Anmeldenummer: 02002598.7
(22) Anmeldetag: 05.02.2002
(51) Int. Cl.: C07C 67/08, C07C 69/704, C07C 69/67

(54) **Verfahren zur Herstellung von acylierten Estern der Zitronensäure**

(30) Priorität: 13.02.2001 DE 10106627
(71) Anmelder: Celanese Chemicals Europe GmbH, 60439 Frankfurt am Main (DE)
(72) Erfinder: Bergrath, Klaus, 46147 Oberhausen (DE); Klein, Thomas, 46149 Oberhausen (DE); Bohnen, Hans, Dr., 47441 Moers (DE)

(57) **Zusammenfassung**

Die vorliegende Erfindung betrifft ein Verfahren zur Herstellung von Estern der Citronensäure aus Citronensäure und linearen oder verzweigten aliphatischen Monoalkoholen mit 4 bis 14 Kohlenstoffatomen im Molekül. Wesentliche Merkmale der neuen Arbeitsweise sind der Einsatz eines begrenzten stöchiometrischen Alkoholüberschusses bezogen auf eingesetzte Säure und die unmittelbare Acylierung des Rohesters, d.h. die Umsetzung des Rohesters mit einem Acylierungsmittel zur Maskierung der OH-Gruppe, ohne dass er zuvor irgendwelchen Reinigungsoperationen unterworfen wurde.

## Beschreibung

Die vorliegende Erfihdung betrifft ein Verfahren zur Herstellung von Estern der Citronensäure mit linearen oder verzweigten aliphatischen C₄- bis C₁₄-Monoalkoholen. Die neue Arbeitsweise ist u.a. durch den Einsatz eines begrenzten stöchiometrischen Alkoholüberschusses bezogen auf eingesetzte Säure charakterisiert. Sie zeichnet sich durch bemerkenswerte verfahrenstechnische Einfachheit aus. Überdies liefert sie sehr reine Produkte, die auch hohen Qualitätsanforderungen, wie sie z.B. bei der Verwendung derartiger Ester als Weichmacher für Kunststoffe gestellt werden, genügen.

Weichmacher finden in großem Umfang Anwendung in Kunststoffen, in Beschichtungsmitteln und Dichtungsmassen sowie in Kautschuk- und Gummiartikeln. Sie treten mit hochpolymeren thermoplastischen Stoffen, ohne chemisch zu reagieren, in physikalische Wechselwirkung, vorzugsweise durch ihr Löse- und Quellvermögen. Hierdurch bildet sich ein homogenes System aus, dessen thermoplastischer Bereich gegenüber den ursprünglichen Polymeren zu niederen Temperaturen verschoben ist. Als Folge des Weichmacher-Zusatzes erhält man u.a. einen Werkstoff, dessen mechanische Eigenschaften gegenüber denen des unbehandelten Ausgangsmaterials optimiert sind. So werden z.B. Formveränderungsvermögen, Elastizität und Festigkeit erhöht und die Härte verringert.

Um Weichmachern eine möglichst weite Anwendung zu erschließen, müssen sie eine Reihe allgemein gültiger Kriterien erfüllen. Im Idealfall sollen sie geruchlos, farblos, licht- und wärmebeständig sein. Überdies verlangt man, dass sie unempfindlich gegenüber Wasser, schwer entflammbar und schwer brennbar und wenig flüchtig sind. Insbesondere im Lebensmittelbereich und für Anwendungen auf medizinischem Gebiet vorgesehene Weichmacher müssen gesundheitlich unbedenklich sein. Schließlich soll die Herstellung der Weichmacher sowohl hinsichtlich der benötigten apparativen Ausrüstung als auch hinsichtlich der erforderlichen Verfahrensschritte einfach sein und, um ökologischen Anforderungen zu genügen, die Bildung nichtverwertbarer Nebenprodukte und schädlicher Abfallstoffe vermeiden.

Aufgrund ihrer ausgezeichneten Weichmachereigenschaften finden bestimmte Phthalate, also Ester der Phthalsäure, umfangreiche Anwendung als Zusatzstoffe für Thermoplaste, insbesondere für PVC. Ihrem universellen Einsatz steht jedoch entgegen, dass diese Ester in der Fachliteratur wiederholt gesundheitlichen Bedenken begegnen. So verbietet sich ihr Einsatz in Verbindung mit Lebensmitteln z.B. als Verpackungsmaterial und in anderen Erzeugnissen, deren Gebrauch aus Gründen der Gesundheitsvorsorge besonderer Sorgfalt unterworfen ist. Zu ihnen gehören z.B. Artikel des täglichen Bedarfs, wie Haushaltsgegenstände und Gegenstände für die Versorgung und Betreuung von Kindern, darin eingeschlossen Spielzeug, sowie Produkte, die im medizinischen Bereich eingesetzt werden. Man verwendet daher als Weichmacher für Hilfs- und Fertigprodukte aus Thermoplasten, die für diese speziellen Anwendungsgebiete bestimmt sind nicht Phthalate sondern die toxisch unbedenklichen Ester der Citronensäure.

Die Herstellung der Citronensäureester erfolgt auf konventionellem Weg durch Reaktion der Säure mit einem Alkohol oder, sofern es die gewünschten Eigenschaften des Fertigprodukts erfordern, mit einem Gemisch unterschiedlicher Alkohole. Die Umsetzung wird mit überschüssigem Alkohol (Molverhältnis Säure:Alkohol >1:3,6) in Gegenwart eines Katalysators durchgeführt, um eine möglichst vollständige Umsetzung der Säure in einer vertretbaren Reaktionszeit sicherzustellen. Der Erzielung eines hohen Umsatzes (entsprechend einer hohen Produktausbeute) kann neben der Verwendung eines Alkoholüberschusses auch die Abtrennung des im Verlauf der Reaktion gebildeten Wassers dienen. Als Katalysatoren bewährt haben sich Säuren wie Schwefelsäure, auch die Verwendung von Titanaten für diesen Zweck wurde beschrieben. Der Estersynthese schließt sich die Entfernung des Katalysators und die Wäsche des Produkts mit Wasser an. Darauf wird der überschüssige Alkohol aus dem Reaktionsgemisch abgetrennt. Anschließend acyliert man die freie OH-Gruppe der Citronensäure, z.B. mit Essigsäure oder Essigsäureanhydrid, zweckmäßigerweise wiederum in Gegenwart eines Katalysators. In einem folgenden Reaktionsschritt werden das Säureanhydrid oder die ursprünglich eingesetzte bzw. aus dem Säureanhydrid gebildete Säure abdestilliert. Es folgt die Neutralisation des Katalysators, die Trocknung des rohen Citronensäureesters und seine Reinigung durch Destillation.

Die aufwendigen Neutralisations-, Wasch- und Destillationsschritte werden als unverzichtbar zur Gewinnung hochwertiger Weichmacher auf Basis von Citronensäureestern angesehen. Hierbei ist zu berücksichtigen, dass Weichmacher eine Anzahl Kriterien erfüllen müssen, um ihnen möglichst eine Vielzahl von Anwendungsgebieten zu eröffnen. So sollen sie, von den bereits erörterten toxikologischen Eigenschaften abgesehen, kälte- und wärmebeständig, unempfindlich gegenüber Wasser, schwer brennbar und wenig flüchtig sein. Diese Eigenschaften werden maßgebend durch die Struktur der Ausgangsstoffe beeinflußt. Darüber hinaus müssen die Weichmacher hohen sensorischen Ansprüchen genügen, d.h. es wird verlangt, dass sie geruchund farblos sind. Diese Eigenschaften stehen in engem Zusammenhang mit der Reinheit der Syntheseprodukte. Bereits in geringster Konzentration (d.h. im ppm-Bereich) im Esterweichmacher enthaltene Verunreinigungen können seine Eignung für die vorgesehene Verwendung in Frage stellen. Obgleich sich Geruch und Farbe durch Zusatz von Additiven den gewünschten Anforderungen anpassen lassen, vermeidet man den Gebrauch von Hilfsstoffen, weil sie andere Eigenschaften der Weichmacher beeinträchtigen können und/oder ihre Einsatzmöglichkeiten, z.B. wegen Unverträglichkeit mit dem Substrat, begrenzen. Schließlich soll die Produktion der Weichmacher einfach sein, also nur wenige, möglichst einfache und apparativ nicht aufwendige Arbeitsschritte umfassen. Überdies wird erwartet, dass das Herstellungsverfahren auch strengen ökologischen Anforderungen genügt und die Entstehung von Abfallstoffen, wie nicht weiterverwertbaren Nebenprodukten und Schadstoffe enthaltenden Abwässern, vermeidet.

Aufgabe der vorliegenden Erfindung ist es ein Verfahren bereitzustellen, das die Herstellung von Esterweichmachern hoher Reinheit auf Basis von Citronensäure in hohen Ausbeuten erlaubt. Besonderer Wert wird in diesem Zusammenhang darauf gelegt, dass der Gesamtprozess nur wenige Einzelschritte umfasst, mit einfachen apparativen Mitteln realisiert werden kann, lange Betriebszeiten sicherstellt und über die Betriebsdauer gleichbleibende Produkte einwandfreier Qualität liefert.

Die Erfindung besteht in einem Verfahren zur Herstellung von Estern der Citronensäure durch Umsetzung von Citronensäure mit aliphatischen Alkoholen im molaren Überschuss in Gegenwart eines Katalysators. Das Verfahren ist dadurch gekennzeichnet, dass man je mol Citronensäure bis zu 3,4 mol eines linearen oder verzweigten Monoalkohols mit 4 bis 14 Kohlenstoffatomen im Molekül einsetzt, die Veresterung bei Temperaturen von 110 bis 140°C in Gegenwart eines sauren Katalysators und eines Schleppmittels zur Entfernung des im Verlauf der Reaktion gebildeten Wassers als azeotropes Gemisch vornimmt, das Reaktionsgemisch unmittelbar darauf bei Temperaturen bis 110°C acyliert, überschüssiges Acylierungsmittel als solches oder als Folgeprodukt abdestilliert, anschließend den sauren Katalysator neutralisiert, das Reaktionsgemisch mit Wasser wäscht, flüchtige Anteile aus dem Gemisch durch Destillation entfernt und den Destillationsrückstand trocknet.

Die neue Arbeitsweise zeichnet sich durch große Zuverlässigkeit nicht nur im Labor- und Versuchsbetrieb sondern auch in technischen Anlagen aus. Sie ist diskontinuierlich wie kontinuierlich leicht durchzuführen und liefert Citronensäureester hoher Reinheit, die verschiedenartige Anwendung, insbesondere auch als Weichmacher finden können. Für den zuletzt genannten Einsatz sind die ausgezeichneten Farbeigenschaften, vor allem die bemerkenswerte Farbstabilität hervorzuheben.

Ein sehr wichtiges Merkmal des erfindungsgemäßen Verfahrens ist die Anwendung eines molaren Alkoholüberschusses in der Veresterungsreaktion und insbesondere die Einhaltung eines Maximalwertes von 3,4 mol Alkohol je mol Citronensäure. Die Limitierung der Alkoholmenge erlaubt es, den Hydroxyester der Citronensäure sofort zu acylieren und auf eine Reihe Verfahrensschritte, hierzu zählen Neutralisation, Wäsche und Destillation im Anschluss an die Veresterung, zu verzichten, deren Einhaltung nach den Verfahren des Standes der Technik unerlässlich für die Gewinnung hochreiner Ester ist. Die Begrenzung des Alkoholüberschusses vermeidet überdies das Auftreten von Problemen, die mit der Umsetzung nunmehr entbehrlicher Arbeitsschritte verbunden waren, z.B. die lästige Schaumbildung während der Wäsche und, im Anschluss daran, die in bestimmten Fällen, z.B. bei der Herstellung von Tributylcitrat auftretende Umkehr von wässriger und organischer Phase, d.h. die Abscheidung der wässrigen oberhalb der organischen Phase. Von besonderer Bedeutung ist auch die Verminderung thermischer Verfahrensschritte. Denn es ist bekannt, dass die thermische Belastung des Ester-Rohproduktes zu Spaltungsprodukten führt, die den Ester nachhaltig kontaminieren und aufwendige Reinigungsmaßnahmen notwendig machen.

Nach einer bevorzugten Ausführungsform der erfindungsgemäßen Arbeitsweise setzt man je mol Citronensäure 3.1 bis 3.35 mol Monoalkohol ein, besonders bewährt hat es sich je mol Citronensäure 3,2 bis 3,3 mol Monoalkohol anzuwenden.

Als Alkoholkomponente zur Herstellung von Estern nach dem neuen Prozess eignen sich sowohl lineare als auch einfach oder mehrfach verzweigte Alkohole mit 4 bis 14 Kohlenstoffatomen im Molekül beliebiger Provenienz. Wegen ihrer leichten Verfügbarkeit finden insbesondere Oxoalkohole Anwendung, d.h. Alkohole die durch Oxosynthese, also Umsetzung von Monoolefinen mit Kohlenmonoxid und Wasserstoff hergestellt wurden. Statt einheitlicher Alkohole können auch Alkoholgemische, d.h. Gemische strukturisomerer Verbindungen oder Gemische von Alkoholen unterschiedlicher Molekülgröße, als Veresterungspartner eingesetzt werden, sofern auf diesem Wege das erwünschte Eigenschaftsbild des Endprodukts eingestellt werden kann. Vorzugsweise enthalten die Monoalkohole, insbesondere wenn sie zur Herstellung von Esterweichmachern dienen, 4 bis 10 Kohlenstoffatome im Molekül, unter diesen haben besondere Bedeutung die Butanole, Hexanole und Octanole.

Als Temperatur, bei der die Umsetzung von Citronensäure und Alkohol vorgenommen wird, hat sich der Bereich von 110 bis 140°C bewährt. Niedrigere Temperaturen sind nicht ausgeschlossen, sofern auf Grund der Eigenart der Reaktionspartner oder der Reaktionsumstände eine genügend hohe Reaktionsgeschwindigkeit erzielt wird oder nur Teilumsätze angestrebt werden. Höhere Temperaturen vermeidet man im allgemeinen um das Risiko der Zersetzung von Ausgangsstoffen, Neben- und Endprodukten und damit der Verunreinigung des Esters, z.B. durch farbschädigende Stoffe, vorzubeugen. Die Anwendung von vermindertem oder erhöhtem Druck während der Reaktion ist möglich, eine solche Ausgestaltung des Verfahrens wird sich jedoch auf Sonderfälle beschränken.

Um unter wirtschaftlichen Aspekten vertretbare Reaktionszeiten sicherzustellen ist es erforderlich, die Geschwindigkeit der Umsetzung von Säure und Alkohohol durch Zusatz eines Katalysators zu erhöhen. Geeignet sind für diesen Zweck die üblichen, katalytisch wirksamen Substanzen wie Schwefelsäure, Ameisensäure, Polyphosphorsäure, Methansulfonsäure oder p-Toluolsulfonsäure, die im Reaktionsgemisch gelöst oder suspendiert als reine Verbindung oder auch in Form eines Gemisches verschiedener Substanzen eingesetzt werden. Bevorzugt werden Schwefelsäure, Methansulfonsäure und p-Toluolsulfonsäure, die, in der angewandten Konzentration, gegenüber Reaktanten und Produkt chemisch indifferent sind, preiswert zur Verfügung stehen und aus dem Reaktionsgemisch leicht entfernt werden können. Die Menge des eingesetzten Katalysators kann sich über einen weiten Bereich erstrecken. Es können z.B. sowohl 0,01 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,01 bis 1,0, vorzugsweise 0,01 bis 0,5 Gew.-%, jeweils bezogen auf das Reaktionsgemisch.

Die Entfernung des Reaktionswassers aus dem Reaktionsgemisch, die insbesondere im Hinblick auf den begrenzten Alkoholüberschuss erforderlich ist, um das Veresterungsgleichgewicht zu Gunsten des Esters zu verschieben, erfolgt mit Hilfe von Azeotropbildnern (Schleppmittel). Üblicherweise wählt man hierfür organische Lösemittel aus, die mit Wasser entsprechende, im Bereich der Reaktionstemperatur von 110 bis 140°C siedende, Mischungen bilden. Beispiele für geeignete Schleppmittel sind Hexan, Cyclohexan, Toluol und die isomeren Xylole, bevorzugt wird Cyclohexan. Die zur vollständigen Abtrennung des Wassers erforderliche Menge Schleppmittel läßt sich aus der, entsprechend der Stöchiometrie der Veresterungsreaktion berechneten Wasserbildung und aus der Zusammensetzung des binären Azeotrops in einfacher Weise ermitteln. Es hat sich bewährt, das Schleppmittel im Überschuss einzusetzen, zweckmäßig in einem Anteil, der 50 bis 200 Gew-% über der theoretisch berechneten Menge liegt. Durch Auffangen und Auftrennen des abdestillierten Schleppmittel-/Wassergemischs lässt sich in einfacher Weise der Fortgang der Reaktion verfolgen. Das aus dem Azeotrop abgeschiedene Schleppmittel kann unmittelbar in die Reaktion zurückgeführt werden.

Die durch Umsetzung von Citronensäure und Alkohol gewonnenen Triester enthaltenen noch eine freie Hydroxylgruppe. Sie muss maskiert werden, um zu verhindern, dass der Ester unerwünschte Reaktionen eingeht. Gebräuchlich ist es diese sehr reaktionsfähige funktionelle Gruppe zu acylieren, d.h. mit einer Säure zu verestern. Als Acylierungsmittel hat sich Essigsäureanhydrid und Buttersäureanhydrid sehr bewährt, doch ist die Verwendung anderer Säuren bzw. Säureanhydride nicht ausgeschlossen. Die Veresterung der OH-Gruppe erfolgt mit Säure bzw. Säureanhydrid, die, bezogen auf den Triester, im Überschuss eingesetzt werden. Es ist vorteilhaft, je mol Triester 1,2 bis 1,6 mol vzw.1,3 bis 1,5 mol einer Monocarbonsäure (bzw. die entsprechende Menge eines Säureanhydrids) anzuwenden. Zweckmässig hält man Reaktionstemperaturen bis 110°C ein, wobei die jeweils auszuwählende aktuelle Temperatur sich nach der Reaktivität des Esters und des Acylierungsmittels richtet. Bevorzugt wird der Temperaturbereich von 60 bis 80°C. Die Reaktion erfolgt in Gegenwart eines Katalysators. Im allgemeinen ist es nicht erforderlich dem Reaktionsgemisch eigens einen Katalysator zuzusetzen, weil es von der Bildung des Triesters Katalysator noch in ausreichender Menge enthält, jedoch ist in Sonderfällen die Zugabe eines Katalysators nicht ausgeschlossen.

Beide Reaktionen, Veresterung der Citronensäure und Acylierung der Hydroxylgruppe, werden in Abwesenheit eines Lösemittels durchgeführt. Es ist jedoch auch möglich in Gegenwart eines Lösemittels zu arbeiten, falls es die individuellen Umstände erfordern oder nahelegen. Häufig erfüllt auch das Schleppmittel gleichzeitig die Aufgabe des Lösemittels.

Erfindungsgemäß wird erst das aus Veresterung und Acylierung hervorgehende Reaktionsprodukt der Reinigung zugeführt, im Gegensatz zur Arbeitsweise des Standes der Technik, die eigene Reinigungsschritte jeweils nach der Veresterung und nach der Acylierung vorsieht. Im Einzelnen wird aus dem rohen Veresterungs- und Acylierungsgemisch zunächst überschüssiges Acylierungsmittel, also Säure oder Säureanhydrid, sowie durch Umsetzung von Acylierungsmittel und der Alkoholkomponente der Veresterungsreaktion gebildeter Ester abdestilliert. Diese Abtrennung von Reaktanten und Nebenprodukten erfolgt auf konventionellem Weg, besondere Vorkehrungen müssen nicht getroffen werden. Der Destillation schließt sich die Neutralisation des Rohproduktes an. Dieser Arbeitsschritt dient der Entfernung des für Veresterung und Acylierung verwendeten Katalysators. Die Neutralisation erfolgt mit Hilfe alkalischer Reagenzien, z.B. Natriumcarbonat oder Natriumhydroxid, die üblicherweise als wässrige Lösungen eingesetzt und mit der organischen Phase durch Rühren oder auf anderem Wege innig vermischt werden. Anschließend trennt man wässrige und organische Phase, wäscht das Reaktionsgemisch mit Wasser und trocknet es zur Beseitigung letzter Feuchtigkeitsspuren nach konventionellen Verfahren. So kann man z.B. die Wasserreste bei mäßig erhöhten Temperaturen unter vermindertem Druck entfernen oder den Strom eines inerten Gases wie Stickstoff durch den Rückstand leiten.

In dem folgenden Beispiel wird das erfindungsgemäße Verfahren näher beschrieben, die neue Arbeitsweise ist jedoch nicht auf diese eine Ausführungsform beschränkt.

### Beispiel

### Herstellung von Acetyl-tri-butyl-citrat

Die Veresterung der Citronensäure mit Butanol und die sich unmittelbar anschließende Acylierung des Triesters mit Essigsäureanhydrid wird in einem beheizbaren 2 I-Kolben durchgeführt, der mit Rührer, Innenthermometer und Einfüllstutzen ausgestattet ist.

In den Kolben werden 210,14 g (1 mol) Citronensäure-monohydrat, 244,57 g (3,3 mol) n-Butanol sowie 1,0 g Methansulfonsäure und 44,0 g Cyclohexan vorgelegt und solange gerührt, bis eine homogene Lösung entstanden ist. Das Reaktionsgemisch wird anschließend auf 110°C erhitzt und 13 Stunden unter Ausschleusung des Reaktionswassers bei dieser Temperatur gehalten. Man gewinnt 399,2 g Rohester, der unmittelbar, d.h. ohne vorherige Aufbereitung oder Reinigung, mit 122,5 g Essigsäureanhydrid (1,2 mol) und 1,0 g (0,01 mol) Methansulfonsäure versetzt und 2 Stunden auf 60°C erhitzt wird. Das überschüssige Acylierungsmittel wird bei einer Temperatur von etwa 60°C und 1hPa abdestilliert und der verbleibende Ester anschließend neutralisiert. Zur Neutralisation gibt man zum acylierten Rohester 59,2 g Wasser und 80,2 g einer wässrigen Lösung, die 10 Gew.-% NaOH enthält. Nach Trennung der beiden Phasen wird der organische Anteil mit Wasser in einer Menge von etwa 50% seines Gewichts gewaschen, darauf durch Phasentrennung von der Hauptmenge des Wassers befreit und schließlich durch 2 Stunden Erhitzen auf maximal 110°C bei etwa 1 hPa Druck getrocknet. Man erhält 390,0 g farblosen, wasserklaren Reinester.

## Patentansprüche

1. Verfahren zur Herstellung von Estern der Citronensäure durch Umsetzung von Citronensäure mit aliphatischen Alkoholen im molaren Überschuss in Gegenwart eines Katalysators **dadurch gekennzeichnet, dass** man je mol Citronensäure bis zu 3,4 mol eines linearen oder verzweigten Monoalkohols mit 4 bis 14 Kohlenstoffatomen im Molekül oder eines Gemisches solcher Alkohole einsetzt, die Veresterung bei Temperaturen von 110 bis 140°C in Gegenwart eines sauren Katalysators und eines Schleppmittels zur Entfernung des im Verlauf der Reaktion gebildeten Wassers als azeotropes Gemisch vornimmt, das Reaktionsgemisch unmittelbar darauf bei Temperaturen bis 110°C acyliert, überschüssiges Acylierungsmittel als solches oder als Folgeprodukt abdestilliert, anschließend den sauren Katalysator neutralisiert, das Reaktionsgemisch mit Wasser wäscht, flüchtige Anteile aus dem Gemisch durch Destillation entfernt und den Destillationsrückstand trocknet.

2. Verfahren nach Anspruch 1 **dadurch gekennzeichnet, dass** man je mol Citronensäure 3,1 bis 3,35, vorzugsweise 3,2 bis 3,3 mol eines linearen oder verzweigten Monoalkohols einsetzt.

3. Verfahren nach Anspruch 1 oder 2 **dadurch gekennzeichnet, dass** der lineare oder verzweigte Monoalkohol 4 bis 10 Kohlenstoffatome im Molekül enthält.

4. Verfahren nach Anspruch 3 **dadurch gekennzeichnet, dass** der lineare oder verzweigte Monoalkohol ein Butanol oder ein Gemisch isomerer Butanole, ein Hexanol oder ein Gemisch isomerer Hexanole oder ein Octanol oder ein Gemisch isomerer Octanole ist.

5. Verfahren nach einem oder mehreren der Ansprüche 1 bis 4 **dadurch gekennzeichnet, dass** die Konzentration des Katalysators bei der Veresterung der Citronensäure mit dem Monoalkohol 0,01 bis 1,0 Gew.-%, vorzugsweise 0,01 bis 0,5 Gew.-% bezogen auf das Reaktionsgemisch beträgt.

6. Verfahren nach einem oder mehreren der Ansprüche 1 bis 5 **dadurch gekennzeichnet, dass** als Veresterungskatalysator Schwefelsäure, Methansulfonsäure oder p-Toluolsulfonsäure eingesetzt wird.

7. Verfahren nach einem oder mehreren der Ansprüche 1 bis 6 **dadurch gekennzeichnet, dass** als Schleppmittel zur Entfernung des, im Verlauf der Veresterung gebildeten Wassers Cyclohexan, Toluol oder eines der isomeren Xylole verwendet wird.

8. Verfahren nach einem oder mehreren der Ansprüche 1 bis 7 **dadurch gekennzeichnet, dass** zur Acylierung je mol Citronensäuretriester 1,2 bis 1,6 mol, vorzugsweise 1,3 bis 1,5 mol Monocarbonsäure bzw. die entsprechende Menge des Anhydrids einer Monocarbonsäure angewendet werden.

9. Verfahren nach einem oder mehreren der Ansprüche 1 bis 8 **dadurch gekennzeichnet, dass** die Acylierung bei Temperaturen von 60 bis 80°C erfolgt.

10. Verfahren nach einem oder mehreren der Ansprüche 1 bis 9 **dadurch gekennzeichnet, dass** das Acylierungsmittel Essigsäureanhydrid oder Buttersäureanhydrid ist.
